Europäisches Patentamt

European Patent Office

Office européen des brevets

(19)

(11) Numéro de publication: **0 207 041**
A2

# (12) DEMANDE DE BREVET EUROPEEN

(21) Numéro de dépôt: 86870080.8

(22) Date de dépôt: 05.06.86

(51) Int. Cl.⁴: **A 61 K 9/20,** A 61 K 9/50,
A 61 K 9/52

(30) Priorité: **12.06.85 LU 85943**

(43) Date de publication de la demande: **30.12.86**
**Bulletin 86/52**

(84) Etats contractants désignés: **AT BE CH DE FR GB IT LI
NL SE**

(71) Demandeur: **"GALEPHAR", rue de la Pastorale, 26-28,
Molenbeek (BE)**

(72) Inventeur: **Deboeck, Arthur Marie, Steenweg op
Edingen 61 A, B-1540 Herne (BE)**
Inventeur: **Fossion, Jacques Jean, Rue du Cours
d'Eau 18, B-1428 Lillois (BE)**
Inventeur: **Brusselman, Joseph Yvon, Avenue du
Cimetière de Bruxelles 106, B-1140 Bruxelles (BE)**
Inventeur: **Baudier, Philippe Raymond, Avenue
Blücher 10, B-1410 Waterloo (BE)**

(74) Mandataire: **Schmitz, Yvon et al, Bureau Gevers
S.A. 7, rue de Livourne Bte 1, B-1050 Bruxelles (BE)**

(54) Comprimés pharmaceutiques permettant l'administration aisée de pellets, leur préparation et leur utilisation.

(57) Comprimé pharmaceutique renfermant des pellets, du type monocouche, rapidement désintégrable, chacun des pellets comportant au moins un principe actif en association avec au moins un excipient pharmaceutiquement acceptable, comprenant un film d'enrobage protecteur extérieur, fortement lubrifié, non collant, de manière à pouvoir libérer le pellet suffisamment rapidement lorsque le comprimé qui renferme les pellets est mis en contact avec un liquide aqueux, même de très faible volume.

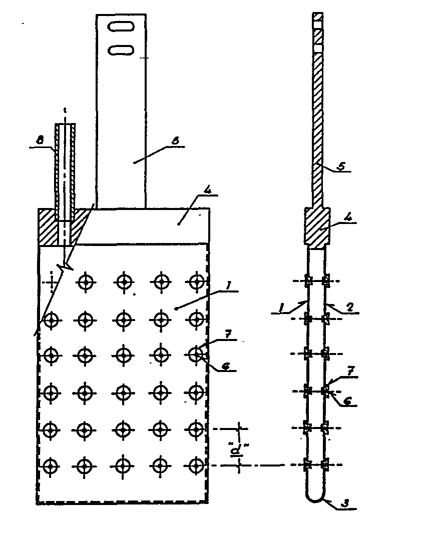

**"Comprimés pharmaceutiques permettant l'administration aisée de pellets, leur préparation et leur utilisation".**

La présente invention est relative à des comprimés pharmaceutiques à administrer par voie buccale, permettant l'administration aisée de substances médicamenteuses lorsqu'elles sont présentées en pellets ou microgranules, à leur préparation et à leur utilisation.

La présente invention concerne plus particulièrement des comprimés comprenant des pellets, qui présentent la caractéristique de libérer ces pellets en un temps très bref, après qu'ils aient été mis en contact avec une certaine quantité, même très petite, et de l'ordre de quelques millilitres, d'un liquide aqueux.

Il est actuellement admis en médecine que l'administration d'un principe actif sous forme de pellets ou microgranules permet dans beaucoup de cas l'optimalisation de l'effet thérapeutique grâce, notamment, à la meilleure répartition de ces pellets dans le tractus digestif par rapport à un comprimé entier [H. Bechgaard, A/S Alfred Benzon, Copenhagen V - Acta Pharmaceutica Technologica 28 (2) 1982)].

Actuellement, lorsqu'un médicament est présenté sous forme de pellets, ceux-ci sont renfermés dans des gélules, ce qui rend impossible pour l'utilisateur le fractionnement de la dose et oblige, par conséquent, le producteur à préparer autant d'unités de prises qu'il y a de dosages nécessaires pour la thérapeutique. Cette présentation en gélules ne facilite cependant pas l'administration des médicaments chez les personnes (enfants, adultes, vieillards) qui éprouvent des difficultés à avaler des préparations solides d'une certaine taille.

Estevenel, Thely et Coulon (brevet des Etats-Unis d'Amérique n°3.922.338) ont mis au point un procédé permettant la compression de pellets sans rupture de ceux-ci, grâce à une technique de compression en triple couche car, disent-ils "la compression directe des microcapsules, qu'elles soient ou non de type enrobé, a pour effet de détruire la structure interne et externe des capsules, et les comprimés ainsi obtenus perdent les propriétés de ces microcapsules. Le rapport entre l'épaisseur de la somme des deux couches extérieures et celle de la couche intérieure est compris de préférence entre 1 et 2,4". Mais le temps de désintégration de ce type de comprimé est long, puisque, comme indiqué, il est de l'ordre de 35 minutes au moins.

La réalisation de tels comprimés à triple couche nécessite un appareillage spécial, coûteux et de réglage difficile. De plus, comme spécifié dans ce brevet, la quantité de pellets actifs qu'il est possible d'incorporer est très faible vu le rapport de hauteur des différentes couches.

Comme on vient de le préciser ci-dessus, les formes médicamenteuses constituées de pellets sont, par conséquent, dosées soit dans des capsules de gélatine dure, soit dans des sachets renfermant la dose unitaire, soit sous forme de comprimés multicouches. Toute autre présentation envisageable, telle que des pellets "en vrac" contenus dans des flacons présente l'inconvénient de rendre peu précis le dosage par le patient lui-même à l'aide d'un quelconque système de mesure.

La présente invention a pour but de remédier aux inconvénients de la technique antérieure, en prévoyant des comprimés en couche unique, sécables ou non, renfermant des pellets qui peuvent être libérés, en un temps très court, lorsque ces comprimés sont mis en contact avec une quantité même très faible de liquide aqueux, l'intégrité de la structure et des propriétés des pellets étant de plus conservée lors de la phase de fabrication proprement dite du comprimé, c'est-à-dire lors de la phase de compression.

A cet effet, suivant l'invention, le comprimé est du type monocouche, rapidement désintégrable et chacun des pellets qu'il renferme, comportant au moins un principe actif en association avec au moins un excipient pharmaceutiquement acceptables, comprend un film d'enrobage protecteur extérieur, fortement lubrifié, non collant, de manière à pouvoir libérer le pellet suffisamment rapidement lorsque le comprimé qui renferme les pellets est mis en contact avec un liquide aqueux, même de très faible volume.

Avantageusement, le film d'enrobage protecteur extérieur des pellets comprend un mélange d'un polymère du type acrylique ou cellulosique et d'un lubrifiant, le polymère du type cellulosique étant de préférence choisi dans le groupe comprenant la carboxyméthylcellulose de sodium, l'éthylcellulose, la méthylcellulose, l'hydroxyéthylcellulose, l'hydroxypropylcellulose, l'hydroxypropylméthylcellulose, l'hydroxybutylméthylcellulose et les mélanges d'au moins deux de ces substances, le lubrifiant étant de préférence choisi dans le groupe comprenant l'acide stéarique, les stéarates d'aluminium, de calcium, de magnésium et de zinc et le talc.

Suivant une forme de réalisation particulièrement avantageuse de l'invention, la teneur en lubrifiant par rapport à la teneur en polymère du type acrylique ou cellulosique du film d'enrobage extérieur des pellets est de l'ordre de 5 à 200% en poids, et de préférence de l'ordre de 10 à 150% en poids.

Suivant une autre forme de réalisation particulièrement avantageuse de l'invention, les pellets comportent chacun au moins un film d'enrobage intérieur permettant de modifier la cinétique de libération du principe actif qu'ils contiennent, ce film d'enrobage intérieur comprenant un ou plusieurs polymères choisis dans le groupe comprenant les polymères du type acrylique , les polymères du type méthacrylique, les polymères du type cellulosique et les polymères du type polyvinylpyrrolidone.

Suivant un mode de réalisation particulièrement avantageux de l'invention, l'enrobage protecteur extérieur de chaque pellet constitue de 1 à 75% en poids, et de préférence de 5 à 50 % en poids du poids de ce pellet.

L'invention se rapporte également à la préparation de ces comprimés pharmaceutiques ainsi qu'à leur méthode d'utilisation, qui consiste à administrer par la voie buccale ledit comprimé à des doses thérapeutiquement efficaces, en entier ou sous forme d'un ou de plusieurs de ses fragments.

Ainsi qu'on l'a déjà mentionné précédemment, le comprimé de l'invention est apte à libérer les pellets qu'il renferme en un temps extrêmement court et dans quelques millilitres de liquide aqueux seulement, comme par exemple le contenu d'une cuillère à café ou à soupe. Ceci laisse donc le choix à l'utilisateur du médicament soit de l'absorber tel quel sous sa forme de comprimé, soit sous la forme buvable des pellets dans le liquide utilisé pour obtenir le délitage du comprimé.

L'absorption sous cette forme de microgranules mélangés à un peu de liquide rend possible l'administration aisée du médicament chez de nombreuses personnes : enfants, adultes, vieillards qui éprouvent des difficultés parfois insurmontables à avaler des médicaments sous forme de comprimés, dragées, gélules, capsules ou autres, lorsque la dissolution, la pulvérisation ou le délitage de ces formes s'avère difficile , impossible voire interdit. Cette technique réunit donc les avantages du dosage précis des principes actifs grâce à la présentation en comprimés et de la déglutition facilitée grâce à la forme microgranules.

On sait que la structure des microgranules est relativement fragile; toutefois, les pellets libérés par délitage du comprimé de l'invention conservent, de façon sensiblement non modifiée , leurs caractéristiques d'avant la compression. Toutefois, le comprimé obtenu doit posséder une dureté suffisante pour qu'il ne s'effrite pas au cours des diverses manipulations de fabrication et de conditionnement ainsi que de transport.

Ainsi qu'on l'a déjà mentionné précédemment, un des inconvénients majeurs des formes galéniques à base de pellets actuellement disponibles sur le marché est l'impossibilité de fractionner la dose pour l'adapter aux besoins du patient, ce qui d'une part oblige le fabricant à mettre à la disposition du corps médical plusieurs conditionnements unitaires différents et d'autre part limite grandement l'utilisation de tels médicaments en pédiatrie, vu le grand nombre de dosages qui seraient rendus nécessaires pour adapter la posologie aux différences et aux variations d'âges et de poids caractéristiques de l'enfance.

Afin de pallier ce problème important, on prévoit également suivant la présente invention des comprimés de pellets sécables en plusieurs fragments suivant des guides gravés, dont les diverses fractions possèdent les mêmes propriétés qualitatives que celles du comprimé entier, et dont chaque fragment contient en outre une quantité connue de substance active.

Les pellets utilisés pour l'obtention des comprimés de la présente invention peuvent présenter des compositions et des caractéristiques de libération de la substance active différentes. Ils peuvent être constitués d'un ou de plusieurs principes actifs et d'un ou de plusieurs excipients choisis parmi des diluants, des mouillants, des densifiants, des acides, des bases, des émulsifiants, des désintégrants ou toute autre substance pharmaceutiquement acceptable.

Afin de masquer, si besoin est, le goût de certaines substances et/ou de résoudre des problèmes d'incompatibilité de principes actifs entre eux ou de principe actif-excipient ou dans tout autre but, les pellets utilisés peuvent être enrobés de façon à leur conférer par exemple des propriétés de libération rapide dans l'estomac, de libération entérique et/ou prolongée dans le temps, du ou des principes actifs qu'ils contiennent.

Un mélange de pellets dont la composition varie soit par le ou les principes actifs qu'ils contiennent, soit par un ou plusieurs des excipients qu'ils renferment, soit par la nature de l'enrobage utilisé, soit par des caractéristiques différentes de libération d'un ou plusieurs principes actifs, peut être utilisé pour la mise sous forme de comprimés suivant la présente invention.

Les pellets utilisés dans la présente invention peuvent être obtenus par extrusion et sphéronisation subséquente d'un mélange plastique formé par le ou les excipients, le ou les principes actifs et le solvant ou le mélange de solvants de granulation.

Pour l'extrusion, divers types d'appareillage, tels que l'ALEXANDERWERK (Alexanderwerk, Allemagne) ou le XTRUDER (Fuji Paudal, Japon) peuvent être utilisés, et le diamètre des trous des filières ou tamis au travers desquels la pâte est extrudée doit être compris entre 0,1 mm et 2 mm, préférentiellement entre 0,5 mm et 1 mm.

Les petits cylindres obtenus après extrusion sont ensuite rendus sphériques au moyen, par exemple, d'un sphéroniseur de type CALEVA (GB) ou MARUMERIZER (Japon).

Les pellets peuvent également être obtenus par des techniques différentes, telles que pistolage et/ou saupoudrage de noyaux appropriés dans des turbines à dragéifier ou au moyen, par exemple, du CF Granulator System de la Firme Freund Industrial Co (Japon) ou encore par simple mélange dans un mélangeur de type planétaire.

Les pellets ainsi obtenus sont séchés par un moyen quelconque tel que, par exemple, une étuve ou un lit fluidisé et ensuite calibrés au diamètre voulu au travers de tamis appropriés.

Les pellets de la présente invention peuvent avoir un diamètre compris entre 0,05 mm et 2 mm et de préférence entre 0,1 et 0,9 mm.

Les principes actifs qui entrent dans la composition des pellets peuvent être quelconques et leur granulométrie doit être égale ou inférieure à la moitié du diamètre moyen des pellets terminés, nus, c'est-à-dire sans enrobage. Comme exemples de principes actifs, on peut citer des sédatifs gastro-intestinaux tels que le métoclopramide et le bromure de propanthéline, des antiacides tels que l'aluminosilicate triple, l'hydroxyde d'aluminium, le sucralfate et la cimétidine, des anti-inflammatoires tels que la phénylbutazone, l'indométhacine, le naproxène, l'ibuprofène, le flurbiprofène, le diclofenac, la dexaméthasone, la prednisone et la prednisolone, des vasodilatateurs coronariens tels que la trinitrine, le dinitrate d'isosorbide, le 5 mononitrate d'isosorbide et le tétranitrate de pentaérythrityle, des vasodilatateurs périphériques et cérébraux comme le suloctidilum, la vincamine, l'oxalate de naftidrofuryle, le mésylate de co-dergocrine, le cyclandélate, la papavérine et l'acide nicotinique, des anti-infectieux tels que l'érythromycine et ses dérivés, la céphalexine, l'acide nalidixique, le chlorhydrate de tétracycline, l'ampicilline, l'amoxycilline, la flucloxacilline sodique, le mandélate de méthénamine, l'hippurate de méthénamine, des neuroleptiques tels que le fluazépam, le diazépam, le témazépam, l'amitriptyline, la doxépine, le carbonate de lithium, le sulfate de lithium, la chlorpromazine, la thioridazine, la triflupérazine, la fluphénazine, la pipérothiazine, l'halopéridol, le chlorhydrate de maprotiline, l'imipramine et la désipramine, des stimulants du système nerveux central tels que le méthylphénidate, l'éphédrine, l'adrénaline, l'isoprénaline, le sulfate d'amphétamine et le chlorhydrate d'amphétamine, des antihistaminiques comme le dimenhydrinate, la diphénylpyraline, la chlorphénamine et la brophéniramine, des antidiarrhéiques tels que le diphénoxylate, des laxatifs comme le bisacodyl, le sulfate de magnésium, le dioctylsulfosuccinate de sodium, des suppléments nutritionnels tels que l'acide ascorbique, l'alpha-tocophérol, la thiamine et la pyridoxine, des antispasmodiques tels que la dicyclovérine et le diphénoxylate, des médicaments qui influencent le rythme cardiaque tels que le vérapamil, la nifédipine, le dilthiazem, le procaïnamide, le disopyramide, le tosylate de bréthylium, le sulfate de quinidine et le gluconate de quinidine, des médicaments utilisés dans le traitement de l'hypertension artérielle, tels que le chlorhydrate de propranolol, le sulfate de guanéthidine, la méthyldopa, le chlorhydrate d'oxprénolol, le captopril et l'hydralazine, des antimigraineux tels que l'ergotamine, des médicaments qui influencent le coagulabilité sanguine, tels que l'acide epsilonaminocaproïque et le sulfate de protamine, des analgésiques tels que l'acide acétylsalicylique, le paracétamol, le phosphate de codéine, le sulfate de codéine, l'oxycodone, le tartrate de dihydrocodéine, l'oxycodéinone, la morphine, l'héroïne, la nalbuphine, le tartrate de butorphanol, le chlorhydrate de pentazocine, la cyclazacine, la péthidine, la buprénorphine, la scopolamine et l'acide méfénamique, des antiépileptiques tels que la phénytoïne sodique, le valproate de sodium et le valproate de lysine, un myorelaxant tel que le dantrolène sodique, des substances employées dans le traitement du diabète, tels que le tolbutamide, le chlorpropamide, le glucagon et l'insuline, des médicaments utilisés dans le traitement des dysfonctions thyroïdiennes, tels que la triiodothyronine, la thyroxine et le propylthiouracile, des diurétiques tels que le furosémide, la chlortalidone, l'hydrochlorothiazide, la spironolactone et le triamtérène, le myorelaxant utérin ritodrine, des anorexigènes tels que le chlorhydrate de diéthylpropion, des antiasthmatiques tels que l'aminophylline, la théophylline, le salbutamol, le sulfate d'orciprénaline et le sulfate de terbutaline, des expectorants comme la guaiphénésine, des antitussifs comme le dextrométhorphane et la noscapine, des mucorégulateurs tels que la carbocystéine et la N-acétylcystéine de lysine, des décongestionnants tels que la phénylpropanolamine et la pseudoéphédrine, des hypnotiques tels que la dichloralphénazone et le nitrazépam, des antinauséeux tels que le théoclate de prométhazine, des hématopoïétiques tels que le sulfate ferreux, l'acide folique et le gluconate de calcium, des uricosuriques tels que la sulpfinpyrazone, l'allopurinol et le probénécide, des extraits de végétaux tels que, par exemple, des extraits de Prunus Africana, de Valériane, de Crataegus, Belladone, Réglisse, etc.. Toutefois, il est bien entendu que l'invention n'est pas limitée aux médicaments cités ci-dessus à titre d'exemples.

Parmi les excipients utilisables dans la fabrication des pellets suivant la présente invention, on peut citer, par exemple, des diluants, tels que lactose, sucrose, xylitol, poudre de lait, celluloses microcristallines (comme les avicels pH 101, 102, RC 581, CL 611), talc, kaolin, magnésie, des agglutinants, tels que dextrines, carboxyméthylcellulose, carbowax, gélatine, polyvinylpyrrolidone et gommes, des mouillants, tels que laurylsulfate de soude, stéarate et oléate de triéthanolamine, polyéthylèneglycols, des désintégrants, tels qu'amidons et leurs dérivés, aérosil., polyvinylpyrrolidone réticulée, des acidifiants, tels qu'acides citrique et tartrique, des alcalinisants, tels que carbonate de calcium, citrate de sodium, gluconate de calcium, des densifiants, tels que sulfate de baryum, ainsi que toute autre substance pharmaceutiquement acceptable. Toutefois, il est entendu que l'invention n'est pas limitée aux excipients susmentionnés. La granulométrie des excipients utilisés doit être inférieure ou au plus égale à la moitié du diamètre moyen des pellets terminés, nus, c'est-à-dire sans enrobage.

Comme liquide de granulation, on peut utiliser un liquide quelconque et en particulier l'eau, le méthanol, l'éthanol, l'isopropanol, le chlorure de méthylène, le chloroforme, l'acétate d'éthyle, l'acétate de butyle, l'acétone, la méthylisobutylcétone, la méthyléthylcétone, ces liquides étant utilisés seuls ou en mélange de deux ou plusieurs d'entre eux.

Afin de conférer aux pellets ainsi obtenus des propriétés particulières, telles que modification de la cinétique de libération du ou des principes actifs, protection vis-à-vis du milieu extérieur et/ou de certaines portions du tractus digestif, divers films ou enrobages dits "intérieurs" peuvent leur être appliqués. Ces films d'enrobage intérieurs pourront être tels que la cinétique de libération du ou des principes actifs soit rapide, lente ou soutenue et/ou différée dans le temps.

Un film procurant une cinétique de libération rapide dès l'arrivée dans l'estomac peut être utilisé soit pour masquer le goût de certains composants du pellet, soit pour protéger par exemple l'oesophage de l'effet irritant de certains principes actifs, soit pour résoudre un problème d'incompatibilité entre divers types de pellets, soit pour augmenter la stabilité en préservant les pellets des agressions du milieu extérieur. Une cinétique de libération lente ou soutenue du ou des principes actifs peut être utilisée pour obtenir des préparations pharmaceutiques dont l'effet thérapeutique sera prolongé et les prises moins fréquentes; une cinétique différée dans le temps peut être utilisée pour éviter la libération du ou des principes dans certaines portions du tractus digestif, telle que, par exemple, par l'emploi d'un film gastro-résistant. Ces films d'enrobage intérieurs peuvent être déposés sur les pellets au moyen de techniques bien connues, telles que pulvérisation en turbine ou en lit fluidisé.

Ces films peuvent être constitués de polymères ou mélanges de polymères des types acrylique (Eudragit), cellulosique [Méthocel, Klucels, Métolose, dérivés cellulosiques tels que les acétates de cellulose et éthylcellulosiques (Aquacoat, Ethocel)]ou du type polyvinylpyrrolidone tels que les dérivés de polyvinylpyrrolidone (copolymères de vinylpyrrolidone, acétate de vinyle),ou de copolymères d'éthers vinylméthyliques et d'anhydride maléique par exemple, associés à un ou plusieurs plastifiants, tels que la triacétine, le

dibutylsébaçate, les polyéthylènes glycols, le polyvinylpyrrolidone, à un ou plusieurs pigments colorés ou non, tels que l'oxyde de titane et les oxydes de fer, à un ou plusieurs agents antiadhérents, tels que le talc, le stéarate de magnésium, à un ou plusieurs agents anti-mousse, tels que les huiles de silicone, à un ou plusieurs agents mouillants, tels que les tweens, spans, laurylsulfate de soude ainsi qu'à un ou plusieurs agents pharmaceutiquement acceptables et utiles à la réalisation du film et/ou à l'obtention des caractéristiques souhaitées. On pourrait, par exemple, utiliser à titre de film gastro-résistant des substances telles que l'acétophtalate de cellulose, les Eudragits des types L 100 et/ou L 30 D, les acétophtalates de polyvinylpyrrolidone.

Les pellets nus ou déjà enrobés d'un film dit d'enrobage intérieur leur conférant une ou des propriétés particulières susmentionnées sont ensuite recouverts d'un film ou enrobage protecteur extérieur, non collant, constitué par un ou des polymères tels que, par exemple, les acryliques, les cellulosiques (ou dérivés de cellulosiques) comme, par exemple, les hydroxypropylméthylcelluloses (Méthocel, Klucels, Sepifilm 002) la carboxyméthylcellulose de sodium, l'éthylcellulose, la méthylcellulose, l'hydroxyéthylcellulose, l'hydroxypropylcellulose et l'hydroxybutylméthylcellulose, contenant une forte proportion de lubrifiant, ou d'un mélange de lubrifiants, comme, par exemple, le talc, l'acide stéarique ou ses sels d'aluminium, calcium, magnésium, zinc. Le rapport entre la quantité de lubrifiant et de filmogène sec doit être compris entre 5% et 200% et de préférence entre 10% et 150%. Ce film protecteur peut également contenir une ou des substances susceptibles, grâce à leurs propriétés de déformabilité à la pression, de protéger l'intégrité de la structure des pellets et/ou d'agir comme agents de délitage grâce à leurs propriétés de gonflement en présence d'un liquide aqueux. Comme agents de délitage de ce type, on peut utiliser, par exemple, des amidons ou leurs dérivés, des dérivés de la polyvinylpyrrolidone, des celluloses microcristallines colloïdales ou non, des polymères anioniques naturels tels que la gomme adragante, les alginates, l'agar-agar. On pourrait également utiliser des plastifiants, tels que la triacétine, le dibutylphtalate, le dioctylphtalate, le diéthylphtalate, la polyvinylpyrrolidone, les polypropylèneglycols, les polyéthylèneglycols, des pigments, tels que l'oxyde de titane, les oxydes de fer, des produits de charge tels que le sulfate de baryum, le kaolin, des agents antistatiques, tels que l'oxyde d'aluminium colloïdal et des mouillants, tels que les tweens, spans,sucroesters.

Le poids de ce film d'enrobage extérieur constitue entre 1% et 75% et de préférence entre 5% et 50% du poids du pellet.

Les pellets terminés sont ensuite mélangés par tout moyen approprié comme, par exemple, un mélangeur cubique, avec la poudre pour compression, laquelle peut avoir été préalablement granulée, en tout ou en partie et peut, ou non, contenir un ou des principes actifs.

Avantageusement, cette poudre comprend, comme excipient, une ou plusieurs substances choisies dans le groupe comprenant l'amidon, le lactose, le xylose, le xylitol, la cellulose microcristalline, l'hydroxypropylméthylcellulose, la carboxyméthylcellulose de sodium, l'éthylcellulose, la méthylcellulose, l'hydroxyéthylcellulose,l'hydroxypropylcellulose et l'hydroxybutylcellulose. Cette poudre peut également comprendre, suivant l'invention, des désintégrants, tels que la polyvinylpyrrolidone réticulée, les celluloses modifiées, telles que celles connues sous la dénomination de Ac-Di-Sol, les amidons naturels ou modifiés, comme par exemple celui connu sous la dénomination d'Explotab, des lubrifiants, tels que les stéarates de magnésium, d'aluminium, de zinc, l'acide stéarique, les polyéthylènes glycols, des agents d'écoulement, tels que l'aérosil, des agents antistatiques, tels que l'oxyde d'aluminium colloïdal, etc.

Le mélange homogène de pellets et de poudre et/ou de granulé pour compression est introduit dans le dispositif d'alimentation d'une machine à comprimer réglée de telle sorte que les comprimés obtenus possèdent un poids convenable et une dureté qui permettent la manipulation et le transport sans problème.

Les comprimés obtenus peuvent avoir des formes quelconques et posséder, lorsqu'ils sont sécables, des gravures servant de guide au fractionnement.

Les exemples décrits ci-après sont destinés à expliciter davantage la présente invention et ne peuvent en aucun cas la limiter.

Exemple n° 1.

Comprimés de pellets de théophylline à action prolongée.

Dans un mélangeur planétaire, on introduit :

|  |  |
|---|---|
| 5,10 kg | théophylline hydratée |
| 0,15 kg | sucre |
| 0,18 kg | polyvinylpyrrolidone |
| 0,69 kg | avicel pH 102 |
| 0,15 kg | polyéthylèneglycol 6000 |

Après avoir mélangé les poudres pendant 5 minutes, on y ajoute 1,6 litre d'eau et on poursuit le mélange pendant 30 minutes. Le mélange humide ainsi obtenu est extrudé dans un appareil XTRUDER muni d'un tamis dont le diamètre des trous est de 0,5 mm et ensuite sphéronisé dans un sphéroniseur CALEVA. Après avoir séché 24 heures dans une étuve chauffée à 60-70°C, les pellets sont enrobés à deux reprises et par fraction de 600 g dans un appareil à lit fluidisé STREA 1 de la firme AEROMATIC.

Enrobage n°1.

pour 600 g de pellets nus

|  |  |
|---|---|
| Stéarate de magnésium | 5,83 g |
| Oxyde de titane | 5,20 g |
| Polyéthylèneglycol 6000 | 8,33 g |
| Eau | 110,32 g |
| Eudragit E 30 D | 199,43 g |

Enrobage n°2.

Sur 500 g de pellets ayant reçu l'enrobage n°1, on pistor, au moyen du même appareillage, la solution suivante :

| | |
|---|---|
| Sepifilm 002 | 43,40 |
| Stéarate de magnésium | 21,75 |
| Eau | 435,03 |

Après séchage pendant 24 heures à l'étuve, on mélange :

| | |
|---|---|
| Pellets enrobés | 400 g |
| Amidon granulé | 400 g |
| Polyvinylpyrrolidone réticulée | 390 g |
| Stéarate de magnésium | 10 g |

Les comprimés sont obtenus par compression au moyen d'une machine à comprimer type 27 de la firme COURTOY.

Caractéristiques des comprimés:

Comprimés cylindriques, divisibles en 4 parties égales

Diamètre    10 mm

Poids    475 mg

Dureté 4,5 à 5,5 Kp

Temps de désagrégation 15 à 20 secondes

MESURE DE LA VITESSE DE LIBERATION DE LA THEOPHYLLINE.

Quantité exprimée en % de théophylline libérée.

| Temps (h) | Avant compression | Après compression |
|---|---|---|
| 1 | 6,36 | 7,33 |
| 2 | 12,19 | 14,60 |
| 4 | 24,30 | 26,99 |
| 6 | 33,96 | 37,14 |
| 8 | 41,20 | 43,09 |
| 10 | 43,99 | 45,20 |

Exemple n° 2.

Comprimés de pellets d'érythromycine base à enrobage entérique.

Les pellets nus sont obtenus en procédant comme dans l'exemple n°1 au départ de

| | |
|---|---|
| Erythromycine base | 7,5 kg |
| Polyvinylpyrrolidone | 0,5 kg |
| Avicel pH 101 | 2,0 kg |
| Eau pour granuler, environ | 3,6 litres |

Les pellets dont la granulométrie est comprise entre 0,1 et 0,8 mm sont ensuite revêtus d'un enrobage entérique constitué du film obtenu par pulvérisation dans une turbine à dragéifier du mélange suivant :

pour 500 g de pellets

| | |
|---|---|
| Acétophtalate de cellulose | 120 g |
| Phtalate de dibutyle | 12 g |
| Acétate d'éthyle | 460 g |
| Ethanol | 440 g |

Après séchage, les pellets sont revêtus d'un second film identique en qualité et en quantité au film n°2 de l'exemple 1.

Les pellets sont ensuite comprimés sur une comprimeuse rotative type R100 (COURTOY) après mélange avec les ingrédients suivants :

| | |
|---|---|
| Pellets | 500 g |
| Lactose granulé | 30 g |
| Amidon granulé | 100 g |
| Ac-Di-Sol | 70 g |
| Stéarate de magnésium | 5 g |
| Arôme cerise | 0,01 g |
| Acide stéarique | 1,5 g |

Caractéristique du comprimé.

Comprimés biconvexes, diamètre 15 mm, sécables en 4 parties égales.

Poids :1250 mg,

Dureté:6 à 6,5 Kp Temps de désagrégation :inférieur à une minute Arôme cerise

Exemple n° 3

Comprimés de pellets de doxycycline à libération "rapide"

En opérant comme dans l'exemple n°1 au départ de :

| | |
|---|---|
| Hyclate de doxycycline | 2,100 kg |
| Méthocel E5 | 0,100 kg |
| Polyvinylpyrrolidone | 0,020 kg |
| Avicel pH 101 | 0,180 kg |
| Laurylsulfate de sodium | 0,030 kg |
| Amidon | 0,100 kg |
| Lactose | 0,450 kg |

Eau, quantité suffisante pour granuler : environ 6 litres.

Les pellets ainsi obtenus, calibrés entre 0,1 mm et 0,9 mm sont ensuite enrobés dans une turbine à dragéifier d'un film à désintégration rapide dans l'estomac et dont la composition est la suivante :

pour 600 g de pellets

| | |
|---|---|
| Solution Eudragit E à 12,5% dans Isopropanol/acétone 60/40 | 400 g |
| Talc | 5 g |
| Stéarate de magnésium | 5 g |
| Isopropanol | 590 g |

Sur les pellets ainsi enrobés, on applique alors le film protecteur dont la formule est identique au film n° 2 de l'exemple n° 1.

Le mélange suivant est ensuite préparé pour être comprimé :

| | |
|---|---|
| Pellets | 600 g |
| Aérosil | 20 g |
| Polyvinylpyrrolidone réticulée | 60 g |

| Explotab | 50 g |
| Amidon granulé | 200 g |
| Polyvinylpyrrolidone | 50 g |
| Stéarate de magnésium | 20g |

La compression se fait alors sur une comprimeuse alternative de type 27 (COURTOY).

Caractéristiques des comprimés obtenus.

Comprimés oblongs divisibles en 2 parties égales Poids: 600 mg Dureté: 5 à 5,5 Kp Temps de désintégration : 30-35 secondes

Exemple n° 4.

Comprimés de pellets d'amoxicilline à libération très rapide.

Les pellets sont fabriqués comme dans l'exemple n°1 au moyen du mélange dont la composition est la suivante :

| Amoxicilline | 8,0 kg |
| Avicel CL 611 | 2,0 kg |
| Eau pour granuler, environ 2,800 litres | |

La fraction de pellets ainsi obtenus dont la granulométrie est comprise entre 0,1 et 0,9 mm de diamètre est ensuite enrobée dans une turbine à dragéifier avec 15% (exprimé en poids sec de film par rapport au poids des pellets) du film suivant :

| Méthocel E5 | 5 parties |
| Talc | 0,5 parties |
| Stéarate de magnésium | 0,5 parties |
| Aquacoat | 5 parties |
| Eau | 89 parties |

Après séchage des pellets, on prépare le mélange suivant pour compression :

| Pellets | 700 g |
| Xylitol | 50 g |
| Lactose granulé | 100 g |
| Ac-Di-Sol | 148 g |
| Polyléthylèneglycol 600 | 2 g |

Les comprimés sont obtenus par compression de la poudre sur une machine à comprimer rotative (COURTOY R100).

Caractéristiques des comprimés.

Comprimés oblongs sécables en 4 parties égales Poids: 1030 mg Dureté 5 à 6 Kp Temps de désintégration : inférieur à 1 minute.

Exemple n° 5.

Comprimés de pellets densifiés d'indométhacine à désagrégation rapide et libération prolongée du principe actif.

1. Fabrication des pellets

Mélanger, dans un mélangeur type planétaire :

| Indométhacine | 1,175 kg |
|---|---|
| Polyvinylpyrrolidone | 1,175 kg |
| Sulfate de baryum | 1,90  kg |

| Avicel pH 101 | 0,75 kg |
|---|---|

Eau pour granuler, environ 1,3 litre

Extruder le mélange humide, sphéroniser les fragments et sécher les pellets à l'étuve à 60°C. Conserver la fraction de pellets comprise entre 0,3 mm et 0,8 mm de diamètre.

2. Enrobage n° 1 des pellets :

Dans un appareil à lit fluidisé, enrober les pellets avec 10% (calculés en poids sec) du film suivant destiné à procurer une libération soutenue du principe actif :

| Stéarate de magnésium | 17,5 g |
|---|---|
| Dioxyde de titane | 10,0 g |
| Polyéthylènegylcol 6000 | 15,0 g |
| Polyvinylpyrrolidone | 15,0 g |
| Indométhacine | 32,5 g |
| Eau | 389,5 g |
| Eudragit E 30 D | 515,5 g |

3. Enrobage n° 2 des pellets

Les pellets recouverts du film n°1 sont enrobés, dans un appareil à lit fluidisé avec 10% (calculés en poids sec) du film protecteur suivant :

| | |
|---|---|
| Hydroxypropylméthylcellulose (SEPIFILM 002) | 65 g |
| Stéarate de magnésium | 20 g |
| Talc | 30 g |
| Amidon de froment | 50 g |
| Polyvinylpyrrolidone | 10 g |
| Eau | 825 g |

4. Compression des pellets

Le mélange suivant est comprimé sur comprimeuse type K27 (COURTOY) :

| | |
|---|---|
| Pellets enrobés 1 + 2 | 600,0 g |
| Avicel pH 102 granulé | 203,0 g |
| Stéarate de magnésium | 4,0 g |
| Polyvinylpyrrolidone réticulée | 116,0 g |
| Aérosil | 1,6 g |

5. Caractéristiques des comprimés obtenus :

Comprimé oblong sécable en 2 parties égales Poids: 600 mg Dureté : 6 - 7 Kp Temps de désagrégation : inférieur à 1 minute

6. Mesure de la cinétique de dissolution de l'indométhacine(méthode de la palette tournante)

| | % d'indométhacine dissoute | |
|---|---|---|
| Temps(h) | Avant compression | Après compression |
| 1 | 3,3 | 4,1 |
| 2 | 6,5 | 7,0 |
| 4 | 18,3 | 17,2 |

| Temps(h) | Avant compression | Après compression |
|---|---|---|
| 6 | 41,7 | 42,3 |
| 8 | 62,0 | 60,8 |
| 10 | 81,5 | 83,0 |

% d' indométhacine dissout (suite)

Il doit être entendu que la présente invention n'est en aucune façon limitée aux formes de réalisation ci-dessus et que bien des modifications peuvent y être apportées sans sortir du cadre du présent brevet.

REVENDICATIONS.

1.    Comprimé pharmaceutique renfermant des pellets, caractérisé en ce qu'il est du type monocouche, rapidement désintégrable et en ce que chacun des pellets, comportant au moins un principe actif en association avec au moins un excipient pharmaceutiquement acceptable , comprend un film d'enrobage protecteur extérieur, fortement lubrifié, non collant, de manière à pouvoir libérer le pellet suffisamment rapidement lorsque le comprimé qui renferme les pellets est mis en contact avec un liquide aqueux, même de très faible volume.

2.    Comprimé suivant la revendication 1, caractérisé en ce que le film d'enrobage protecteur extérieur susdit des pellets comprend un mélange d'un polymère du type acrylique ou cellulosique et d'un lubrifiant.

3.    Comprimé suivant la revendication 2, caractérisé en ce que le polymère du type cellulosique est choisi dans le groupe comprenant la carboxyméthylcellulose de sodium, l'éthylcellulose, la méthylcellulose, l'hydroxyéthylcellulose, l'hydroxypropylcellulose, l'hydroxypropylméthylcellulose, l'hydroxybutylméthylcellulose et les mélanges d'au moins deux de ces substances.

4.    Comprimé suivant l'une ou l'autre des revendications 2 et 3, caractérisé en ce que le lubrifiant est choisi dans le groupe comprenant l'acide stéarique, les stéarates d'aluminium, de calcium, de magnésium et de zinc et le talc.

5.    Comprimé suivant l'une quelconque des revendications 2 à 4, caractérisé en ce que la teneur en lubrifiant par rapport à la teneur en polymère du type acrylique ou cellulosique du film d'enrobage extérieur des pellets est de l'ordre de 5 à 200% en poids.

6.    Comprimé suivant la revendication 5, caractérisé en ce que la teneur  en lubrifiant par rapport à la teneur en polymère du type acrylique ou cellulosique est de l'ordre de 10 à 150% en poids.

7. Comprimé suivan⸱ l'une quelconque des revendications 2 à 6, caractérisé en ce que l⸳ film d'enrobage protecteur extérieur contient au moins un plastifiant, tel que la triacétine, le dibutylphtalate, le dioctylphtalate, le diéthylphtalate, la polyvinylpyrrolidone, les polypropylènes glycols, les polyéthylèneglycols et/ou au moins un agent de délitage, tel que l'amidon, les celluloses microcristalline ou colloïdale et/ou au moins un pigment, tel que l'oxyde de titane, les oxydes de fer et/ou au moins un produit de charge, tel que le sulfate de baryum, le kaolin et/ou un agent antistatique, tel que l'oxyde d'aluminium colloïdal et/ou au moins un mouillant, tel que les tweens,spans, sucroesters.

8. Comprimé suivant l'une quelconque des revendications 1 à 7, caractérisé en ce que chacun des pellets comprend, comme excipient, au moins une substance choisie dans le groupe comprenant le lactose, le sucrose, le xylitol, la poudre de lait, les celluloses microcristallines, le talc, la kaolin, la magnésie, les dextrines, la carboxyméthylcellulose, les carbowax, la gélatine, la polyvinylpyrrolidone, les gommes, le laurylsulfate de soude, les stéarate et oléate de triéthanolamine, les polyéthylèneglycols, les amidons et leurs dérivés, l'aérosil, la polyvinylpyrrolidone réticulée, les acide citrique et tartrique, le carbonate de calcium, le citrate de sodium, le gluconate de sodium et le sulfate de baryum.

9. Comprimé suivant l'une quelconque des revendications 1 à 8, caractérisé en ce que chacun des pellets comprend, comme principe actif, au moins une substance choisie dans le groupe comprenant les sédatifs gastro-intestinaux, les antiacides, les antiinflammatoires, les vasodilatateurs coronaires, les vasodilatateurs périphériques et cérébraux, les anti-infectieux, les neuroleptiques, les stimulants du système nerveux central, les antihistaminiques, les antidiarrhéiques, les laxatifs, les suppléments nutritionnels, les·antispasmodiques, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs les décongestionnants, les hypnotiques, les antinauséeux, les hématopoïétiques, les uricosuriques et les extraits de végétaux.

10. Comprimé suivant l'une quelconque des revendications 1 à 9, caractérisé en ce que les pellets comportent chacun au moins un film d'enrobage intérieur permettant de modifier la cinétique de libération du principe actif qu'ils contiennent.

11. Comprimé suivant la revendication 10, caractérisé en ce que ledit film d'enrobage intérieur est utilisé pour obtenir une libération rapide, lente ou soutenue du principe actif que les pellets contiennent.

12. Comprimé suivant la revendication 10, caractérisé en ce que ledit film d'enrobage intérieur est utilisé pour obtenir une libération différée du principe actif que les pellets contiennent.

13. Comprimé suivant l'une ou l'autre des revendications 11 et 12, caractérisé en ce que le film d'enrobage intérieur comprend au moins un polymère choisi dans le groupe comprenant les polymères du type acrylique, les polymères du type méthacrylique, les polymères du type cellulosique et les polymères du type polyvinylpyrrolidone.

14. Comprimé suivant la revendication 13, caractérisé en ce que le film d'enrobage intérieur susdit comprend au moins une substance choisie parmi les plastifiants, les pigments, les antiadhérents, les agents anti-mousse et les agents mouillants.

15. Comprimé suivant l'une quelconque des revendications 1 à 14, caractérisé en ce que l'enrobage protecteur extérieur de chaque pellet constitue de 1 à 75% en poids du poids de ce pellet.

16. Comprimé suivant la revendication 15, caractérisé en ce que l'enrobage protecteur extérieur du pellet constitue de 5 à 50% en poids du poids de celui-ci.

17. Comprimé suivant l'une quelconque des revendications 1 à 16, caractérisé en ce que les pellets ont un diamètre de l'ordre de 0,05 à 2 mm.

18. Comprimé suivant la revendication 17, caractérisé en ce que le diamètre des pellets est de l'ordre de 0,1 à 0,9 mm.

19. Comprimé suivant l'une ou l'autre des revendications 17 et 18, caractérisé en ce que les principe actif et excipient pharmaceutiquement acceptables des pellets ont une granulométrie qui n'excède pas la moitié du diamètre moyen de ces pellets, non compte tenu de leur enrobage extérieur et de leur éventuel enrobage intérieur.

20. Comprimé suivant l'une quelconque des revendications 1 à 19, caractérisé en ce que le temps de libération des pellets est inférieur à deux minutes.

21. Comprimé suivant l'une quelconque des revendications 1 à 20, caractérisé en ce que le volume de liquide aqueux dans lequel est placé le comprimé pour obtenir sa désagrégation est égal ou supérieur au volume du comprimé lui-même.

22. Comprimé suivant l'une quelconque des revendications 1 à 21, caractérisé en ce qu'il comprend une poudre pour compression comprenant, comme excipient, au moins une substance choisie dans le groupe comprenant les amidons, le lactose, le xylose, le xylitol, la cellulose microcristalline, l'hydroxypropylméthylcellulose, la carboxyméthylcellulose de sodium, l'éthylcellulose, la méthylcellulose, l'hydroxyéthylcellulose, l'hydroxypropylcellulose et l'hydroxybutylméthylcellulose.

0207041

23. Comprimé suivant la revendication 22, caractérisé en ce que la poudre pour compression comprend au moins une substance choisie parmi les désintégrants, les gélifiants, les aromatisants et les colorants.

24. Comprimé suivant l'une ou l'autre des revendications 22 et 23, caractérisé en ce que la poudre pour compression comprend au moins un principe actif.

25. Comprimé suivant l'une quelconque des revendications 1 à 24, caractérisé en ce qu'il comporte sur une de ses faces au moins une rainure servant de guide à son fractionnement.

26. Procédé de préparation du comprimé suivant l'une quelconque des revendications 1 à 25, caractérisé en ce que l'on enrobe chaque pellet du film protecteur extérieur précité, cet enrobage extérieur constituant de 1 à 75%, et de préférence de 5 à 50% en poids du poids du pellet, on mélange la poudre pour compression avec les pellets ainsi enrobés et on comprime le mélange ainsi obtenu pour obtenir un comprimé de poids et de dureté voulus.

27. Procédé suivant la revendication 26, caractérisé en ce que l'on enrobe préalablement chaque pellet du film intérieur précité.

28. Procédé suivant l'une ou l'autre des revendications 26 et 27, caractérisé en ce que l'on granule au moins en partie la poudre pour compression.

29. Méthode d'utilisation du comprimé suivant l'une quelconque des revendications 1 à 25, caractérisée en ce que l'on administre par voie buccale à des doses thérapeutiquement efficaces, en entier ou sous forme d'un ou de plusieurs de ses fragments.

0207041

REVENDICATIONS (AUTRICHE)

1. Procédé de préparation d'un comprimé pharmaceutique renfermant des pellets, caractérisé en ce que l'on enrobe chaque pellet d'un film protecteur extérieur, cet enrobage extérieur constituant de 1 à 75%, et de préférence de 5 à 50% en poids du poids du pellet, on mélange une poudre pour compression avec les pellets ainsi enrobés et on comprime le mélange ainsi obtenu pour obtenir un comprimé de poids et de dureté voulus.

2. Procédé suivant la revendication 1, caractérisé en ce que l'on enrobe préalablement chaque pellet d'un film intérieur.

3. Procédé suivant l'une ou l'autre des revendications 1 et 2, caractérisé en ce que l'on granule au moins en partie la poudre pour compression.

4. Procédé suivant l'une quelconque des revendications 1 à 3, caractérisé en ce que le comprimé pharmaceutique renfermant des pellets est du type monocouche, rapidement désintégrable et en ce que le film d'enrobage protecteur extérieur précité de chacun des pellets comportant au moins un principe actif en association avec au moins une excipient pharmaceutiquement acceptables, est fortement lubrifié, non collant, de manière à pouvoir libérer le pellet suffisamment rapidement lorsque le comprimé qui renferme les pellets est mis en contact avec un liquide aqueux, même de très faible volume.

5. Procédé suivant l'une quelconque des revendications 1 à 4, caractérisé en ce que le film d'enrobage protecteur extérieur susdit des pellets comprend un mélange d'un polymère du type acrylique ou cellulosique et d'un lubrifiant.

6. Procédé suivant la revendication 5, caractérisé en ce que le polymère du type cellulosique est choisi dans le groupe comprenant la carboxyméthylcellulose de sodium, l'éthylcellulose, la méthylcellulose, l'hydroxyéthylcellulose, l'hydroxypropylcellulose, l'hydroxypropylméthylcellulose, l'hydroxybutylméthylcellulose et les mélanges d'au moins deux de ces substances.

7. Procédé suivant l'une ou l'autre des revendications 5 et 6, caractérisé en ce que le lubrifiant est choisi dans le groupe comprenant l'acide stéarique, les stéarates d'aluminium, de calcium, de magnésium et de zinc et le talc.

8. Procédé suivant l'une quelconque des revendications 5 à 7, caractérisé en ce que la teneur en lubrifiant par rapport à la teneur en polymère du type acrylique ou cellulosique du film d'enrobage extérieur des pellets est de l'ordre de 5 à 200% en poids.

9. Procédé suivant la revendication 8, caractérisé en ce que la teneur en lubrifiant par rapport à la teneur en polymère du type acrylique ou cellulosique est de l'ordre de 10 à 150% en poids.

10. Procédé suivant l'une quelconque des revendications 1 à 9, caractérisé en ce que le film d'enrobage protecteur extérieur contient au moins un plastifiant tel que la triacétine, le dibutylphtalate, le dioctylphtalate, le diéthylphtalate, la polyvinylpyrrolidone, les polypropylène glycols, les polyéthylèneglycols et/ou au moins un agent de délitage, tel que l'amidon, les celluloses microcristalline ou colloïdale et/ou au moins un pigment, tel que l'oxyde de titane, les oxydes de fer et/ou au moins un produit de charge, tel que le sulfate de baryum, le kaolin et/ou un agent antistatique, tel que l'oxyde d'aluminium colloïdal et/ou au moins un mouillant, tel que les tweens, spans, sucroesters.

11. Procédé suivant l'une quelconque des revendications 1 à 10, caractérisé en ce que chacun des pellets comprend, comme excipient, au moins une substance choisie dans le groupe comprenant le lactose, le sucrose, le xylitol, la poudre de lait, les celluloses microcristallines, le talc, le kaolin, la magnésie, les dextrines, la carboxyméthylcellulose, les carbowax, la gélatine, la polyvinylpyrrolidone, les gommes, le laurylsulfate de soude, les stéarate et oléate de triéthanolamine, les polyéthylèneglycols, les amidons et leurs dérivés, l'aérosil, la polyvinylpyrrolidone réticulée , les acides citrique et tartrique, le carbonate de calcium, le citrate de sodium, le gluconate de sodium et le sulfate de baryum.

12. Procédé suivant l'une quelconque des revendications 1 à 11, caractérisé en ce que chacun des pellets comprend, comme principe actif, au moins une substance choisie dans le groupe comprenant les sédatifs gastro-intestinaux, les antiacides, les antiinflammatoires, les vasodilatateurs coronaires, les vasodilatateurs périphériques et cérébraux, les anti-infectieux, les neuroleptiques, les stimulants du système nerveux central, les antihistaminiques, les antidiarrhéiques, les laxatifs, les suppléments nutritionnels, les antispasmodiques, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques et les extraits de végétaux.

13. Procédé suivant l'une quelconque des revendications 1 à 12, caractérisé en ce que les pellets comportent chacun au moins un film d'enrobage intérieur permettant de modifier la cinétique de libération du principe actif qu'ils contiennent.

14. Procédé suivant la revendication 13, caractérisé en ce que ledit film d'enrobage intérieur est utilisé pour obtenir une libération rapide, lente ou soutenue du principe actif que les pellets contiennent.

15. Procédé suivant la revendication 13, caractérisé en ce que ledit film d'enrobage intérieur est utilisé pour obtenir une libération différée du principe actif que les pellets contiennent.

16. Procédé suivant l'une ou l'autre des revendications 14 et 15, caractérisé en ce que le film d'enrobage intérieur comprend au moins un polymère choisi dans le groupe comprenant les polymères du type acrylique, les polymères du type méthacrylique, les polymères du type cellulosique et les polymères du type polyvinylpyrrolidone.

17. Procédé suivant la revendication 16, caractérisé en ce que le film d'enrobage intérieur susdit comprend au moins une substance choisie parmi les plastifiants, les pigments, les antiadhérents, les agents anti-mousse et les agents mouillants.

18. Procédé suivant l'une quelconque des revendications 1 à 17, caractérisé en ce que les pellets ont un diamètre de l'ordre de 0,05 à 2 mm.

19. Procédé suivant la revendication 18, caractérisé en ce que le diamètre des pellets est de l'ordre de 0,1 à 0,9 mm.

20. Procédé suivant l'une ou l'autre des revendications 18 et 19, caractérisé en ce que les principes actif et excipient pharmacologiquement acceptables des pellets ont une granulométrie qui n'excède pas la moitié du diamètre moyen de ces pellets, non compte tenu de leur enrobage extérieur et de leur éventuel enrobage intérieur.

21. Procédé suivant l'une quelconque des revendications 1 à 20, caractérisé en ce que le temps de libération des pellets est inférieur à deux minutes.

22. Procédé suivant l'une quelconque des revendications 1 à 21, caractérisé en ce que le volume de liquide aqueux dans lequel est placé le comprimé pour obtenir sa désagrégation est supérieur au volume du comprimé lui-même.

23. Procédé suivant l'une quelconque des revendications 1 à 22, caractérisé en ce que la poudre pour compression précitée comprend, comme excipient, au moins une substance choisie dans le groupe comprenant les amidons, le lactose, le xylose, le xylitol, la cellulose microcristalline, l'hydroxypropylméthylcellulose, la carboxyméthylcellulose de sodium, l'éthylcellulose, la méthylcellulose, l'hydroxyéthylcellulose, l'hydroxypropylcellulose et l'hydroxybutylméthylcellulose.

24. Procédé suivant la revendication 23, caractérisé en ce que la poudre pour compression comprend au moins une substance choisie parmi les désintégrants, les gélifiants, les aromatisants et les colorants.

25. Procédé suivant l'une ou l'autre des revendications 23 et 24, caractérisé en ce que la poudre pour compression comprend au moins un principe actif.

25

0207041

26. Procédé suivant l'une quelconque des revendications 1 à 25, caractérisé en ce que le comprimé pharmaceutique comporte sur une de ses faces au moins une rainure servant de guide à son fractionnement.